(19) Europäisches Patentamt
European Patent Office
Office européen des brevets

(11) Veröffentlichungsnummer: **0 270 971**
**A2**

(12) # EUROPÄISCHE PATENTANMELDUNG

(21) Anmeldenummer: **87117669.9**

(22) Anmeldetag: **30.11.87**

(51) Int. Cl.[4]: **C07C 131/00**, C07C 121/457, C07C 153/05, C07D 307/52, C07D 231/12, A01N 47/40

(30) Priorität: **12.12.86 DE 3642450**

(43) Veröffentlichungstag der Anmeldung: **15.06.88 Patentblatt 88/24**

(84) Benannte Vertragsstaaten: **AT BE CH DE ES FR GB GR IT LI NL SE**

(71) Anmelder: **BAYER AG**
**D-5090 Leverkusen(DE)**

(72) Erfinder: **Gayer, Herbert, Dr.**
**Alfred-Delp-Strasse 4**
**D-4019 Monheim(DE)**
Erfinder: **Jelich, Klaus, Dr.**
**Pahlkestrasse 5**
**D-5600 Wuppertal 1(DE)**
Erfinder: **Krämer, Wolfgang, Dr.**
**Rosenkranz 25**
**D-5093 Burscheid 2(DE)**
Erfinder: **Brandes, Wilhelm, Dr.**
**Eichendorffstrasse 3**
**D-5653 Leichlingen(DE)**

(54) **Substituierte Benzamide.**

(57) Neue substituierte Benzamide der allgemeinen Formel (I),

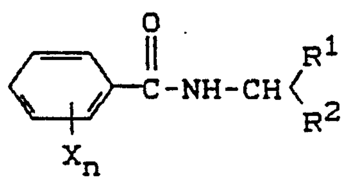 (I)

in welcher
$R^1$ für Alkoxy, Alkenyloxy, Alkinyloxy oder für einen Heterocyclylrest steht,
$R^2$ für Cyano, für einen Carbamoyl-oder für einen Thiocarbamoylrest steht,
X für einen Alkanoyl-oder für einen Alkoximinoalkylrest steht und
n für eine Zahl 1, 2 oder 3 steht,
und deren Verwendung in Schädlingsbekämpfungsmitteln.

Die neuen substituierten Benzamide der Formel (I) können nach Analogieverfahren hergestellt werden, so z.B., indem man geeignete bromsubstituierte Benzamide mit geeigneten Alkoholen oder Heterocyclen umsetzt oder indem man z.B. geeignete Aminoacetonitrile mit geeigneten Alkanoylbenzoylhalogeniden umsetzt, die in einer weiteren Reaktionsstufe z.B. mit geeigneten Hydroxylaminderivaten umgesetzt werden können.

EP 0 270 971 A2

Xerox Copy Centre

BAYER AKTIENGESELLSCHAFT 5090 Leverkusen, Bayerwerk
Konzernverwaltung RP
Patentabteilung Bas/m-c
(Ib)

# Substituierte Benzamide

Die Erfindung betrifft neue substituierte Benzamide, mehrere Verfahren zu ihrer Herstellung und ihre Verwendung als Schädlingsbekämpfungsmittel.

Es ist bereits bekannt, daß bestimmte substituierte Amide, wie beispielsweise das 2-(4-Chlorbenzamido)-2-ethoxy-acetonitril, fungizide und herbizide Eigenschaften besitzen (vgl. z.B. EP 59 536).

Die fungizide Wirksamkeit der vorbekannten Verbindungen ist jedoch insbesondere bei niedrigen Aufwandmengen und Konzentrationen nicht in allen Anwendungsbereichen völlig zufriedenstellend. Hinzu kommt, daß die herbizide Wirkungskomponente der vorbekannten Verbindungen oft nicht genügend selektiv ist und es zu unerwünschten Schäden an den behandelten Kulturpflanzen kommt.

Es wurden neue substituierte Benzamide der allgemeinen Formel (I),

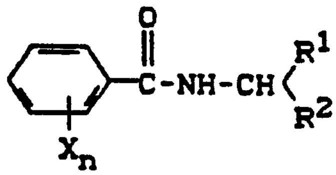 (I)

in welcher

$R^1$ für Alkoxy, Alkenyloxy, Alkinyloxy oder für einen Heterocyclylrest steht,

$R^2$ für Cyano, für einen Carbamoyl- oder für einen Thiocarbamoylrest steht,

X für einen Alkanoyl- oder für einen Alkoximinoalkylrest steht und

n für eine Zahl 1, 2 oder 3 steht,

gefunden.

Weiterhin wurde gefunden, daß man die neuen substituierten Benzamide der allgemeinen Formel (I)

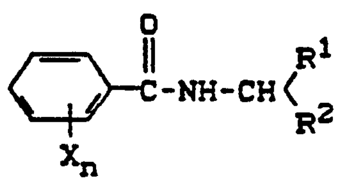 (I)

in welcher

$R^1$ für Alkoxy, Alkenyloxy, Alkinyloxy oder für einen Heterocyclylrest steht,

$R^2$ für Cyano, für einen Carbamoyl- oder für einen Thiocarbamoylrest steht,

X für einen Alkanoyl- oder für einen Alkoximinoalkylrest steht und

n für eine Zahl 1, 2 oder 3 steht,

nach einem der im folgenden beschriebenen Verfahren erhält:

(a) Man erhält substituierte Benzamide der Formel (Ia),

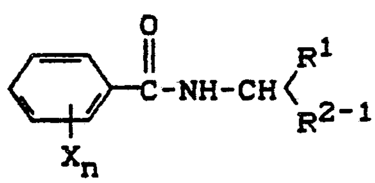

(Ia)

in welcher

$R^1$, X und n die oben angegebene Bedeutung haben und

$R^{2-1}$ für Cyano oder für einen Carbamoylrest steht,

wenn man bromsubstituierte Benzamide der Formel (II),

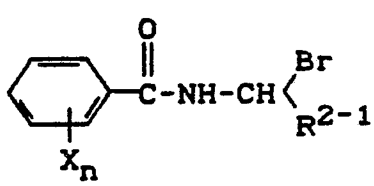

(II)

in welcher

$R^{2-1}$, X und n die oben angegebene Bedeutung haben,

mit Alkoholen oder Heterocyclen der Formel (III)

$R^1$-H (III)

in welcher

$R^1$ die oben angegebene Bedeutung hat,

gegebenenfalls in Gegenwart eines Verdünnungsmittels und gegebenenfalls in Gegenwart eines Säurebindemittels umsetzt;

(b) man erhält substituierte Benzamide der Formel (Ib)

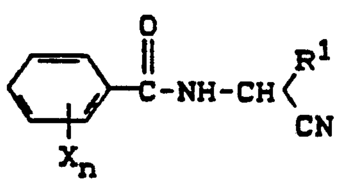

(Ib)

in welcher

$R^1$, X und n die oben angegebene Bedeutung haben,

alternativ auch, wenn man die nach Verfahren (a) erhältlichen substituierten Benzamide der Formel (Ia1)

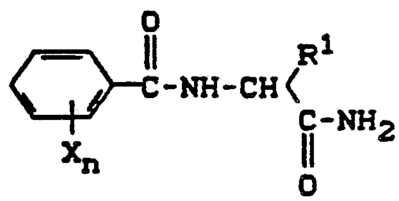 (Ia1)

in welcher

$R^1$, X und n die oben angegebene Bedeutung haben,

gegebenenfalls in Gegenwart eines Verdünnungsmittels und gegebenenfalls in Gegenwart eines Säurebindemittels mit einem wasserabspaltenden Reagenz dehydratisiert;

(c) man erhält substituierte Benzamide der Formel (Ic)

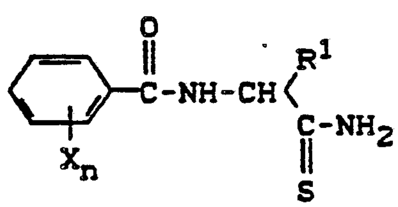 (Ic)

in welcher

$R^1$, X und n die oben angegebene Bedeutung haben,

wenn man die nach Verfahren (a), (b), (d) oder (e) erhältlichen substituierten Benzamide der Formel (Ib)

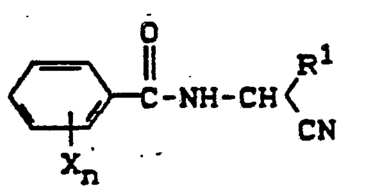 (Ib)

in welcher

$R^1$, X und n die oben angegebene Bedeutung haben,

mit Schwefelwasserstoff, gegebenenfalls in Gegenwart eines Verdünnungsmittels und gegebenenfalls in Gegenwart eines basischen Katalysators umsetzt;

(d) man erhält substituierte Benzamide der Formel (Id)

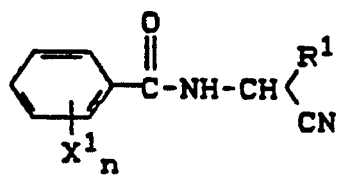

(Id)

in welcher

$R^1$ und n die oben angegebene Bedeutung haben und

$X^1$ für einen Alkoximinoalkylrest steht,

alternativ auch, wenn man die nach Verfahren (a), (b) oder (e) erhältlichen substituierten Benzamide der Formel (If)

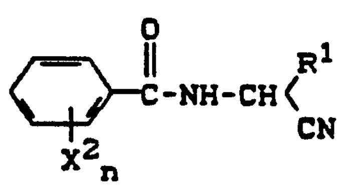

(If)

in welcher

$R^1$ und n die oben angegebene Bedeutung haben und

$X^2$ für einen Alkanoylrest steht,

mit Hydroxylamin-Derivaten der Formel (IV)

$R^3O-NH_2$ (IV)

in welcher

$R^3$ für Alkyl steht,

oder mit deren Säureadditionssalzen,

gegebenenfalls in Gegenwart eines Verdünnungsmittels und gegebenenfalls in Gegenwart eines Säurebindemittels umsetzt;

(e) man erhält substituierte Benzamide der Formel (Ie)

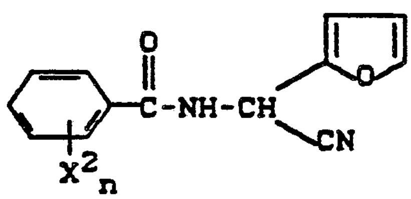

(Ie)

in welcher

$X^2$ und n die oben angegebene Bedeutung haben,

alternativ auch, wenn man substituierte Benzoylchloride der Formel (V),

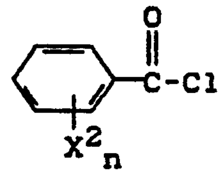

(V)

in welcher

$X^2$ und n die oben angegebene Bedeutung haben,

mit Furanylaminoacetonitril der Formel (VI)

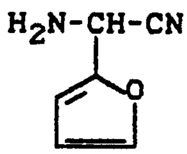

(VI)

oder dessen Säureadditionssalzen gegebenenfalls in Gegenwart eines Verdünnungsmittels und gegebenenfalls in Gegenwart eines Säurebindemittels umsetzt.

Schließlich wurde gefunden, daß die neuen substituierten Benzamide der allgemeinen Formel (I) eine Wirkung gegen Schädlinge besitzen.

Überraschenderweise zeigen die neuen substituierten Benzamide der allgemeinen Formel (I) bei vergleichbar guter oder besserer fungizider Wirkung eine erheblich verbesserte Kulturpflanzenverträglichkeit als die aus dem Stand der Technik bekannten substituierten Amide, wie beispielsweise das 2-(4-Chlorbenzamido)-2-ethoxy-acetonitril, welches chemisch und wirkungsmäßig naheliegende Verbindungen sind.

Die erfindungsgemäßen substituierten Benzamide sind durch die Formel (I) allgemein definiert. Bevorzugt sind Verbindungen der Formel (I), bei welchen

$R^1$ für geradkettiges oder verzweigtes Alkoxy mit 1 bis 8 Kohlenstoffatomen, für jeweils geradkettiges oder verzweigtes Alkenyloxy oder Alkinyloxy mit jeweils 3 bis 8 Kohlenstoffatomen oder für einen 5- oder 6-gliedrigen Heterocyclylrest mit 1 bis 4 Heteroatomen, insbesondere Stickstoff, Sauerstoff und/ oder Schwefel steht,

$R^2$ für Cyano, für einen Carbamoyl- oder für einen Thiocarbamoylrest steht,

X für einen geradkettigen oder verzweigten Alkanoylrest mit 1 bis 6 Kohlenstoffatomen oder für einen geradkettigen oder verzweigten Alkoximinoalkylrest mit jeweils 1 bis 6 Kohlenstoffatomen in den einzelnen Alkylteilen steht und

n für eine Zahl 1, 2 oder 3 steht.

Besonders bevorzugt sind Verbindungen der Formel (I), bei welchen

$R^1$ für geradkettiges oder verzweigtes Alkoxy mit 1 bis 6 Kohlenstoffatomen, für jeweils geradkettiges oder verzweigtes Alkenyloxy oder Alkinyloxy mit jeweils

3 bis 6 Kohlenstoffatomen oder für einen 1-Pyrazolyl-, 1-Imidazolyl-, 1-(1,2,4-Triazolyl)-, 1-Tetrazolyl- oder 2-Furylrest steht,

$R^2$ für Cyano, für einen Carbamoyl- oder für einen Thiocarbamoylrest steht,

X für einen geradkettigen oder verzweigten Alkanoylrest mit 1 bis 4 Kohlenstoffatomen oder für einen geradkettigen oder verzweigten Alkoximinoalkylrest mit jeweils 1 bis 4 Kohlenstoffatomen in den einzelnen Alkylteilen steht und

n für eine Zahl 1 oder 2 steht.

Ganz besonders bevorzugt sind Verbindungen der Formel (I), bei welchen

$R^1$ für Methoxy, Ethoxy, n- oder i-Propoxy, n-, i-, s- oder t-Butoxy, für Allyloxy, n- oder i-Butenyloxy, Propargyloxy, n- oder i-Butinyloxy, für einen 2-Furylrest oder für einen 1-Pyrazolylrest steht,

$R^2$ für Cyano steht,

X für Formyl, Acetyl oder Methoximinomethyl steht und

n für 1 steht.

Im einzelnen sei auf die als Herstellungbeispiele genannten Verbindungen verwiesen.

Verwendet man beispielsweise N-(Brom-cyano-methyl)-4-methoximinomethyl-benzamid und Ethanol als Ausgangsstoffe, so läßt sich der Reaktionsablauf des erfindungsgemäßen Verfahrens (a) durch das folgende Formelschema darstellen:

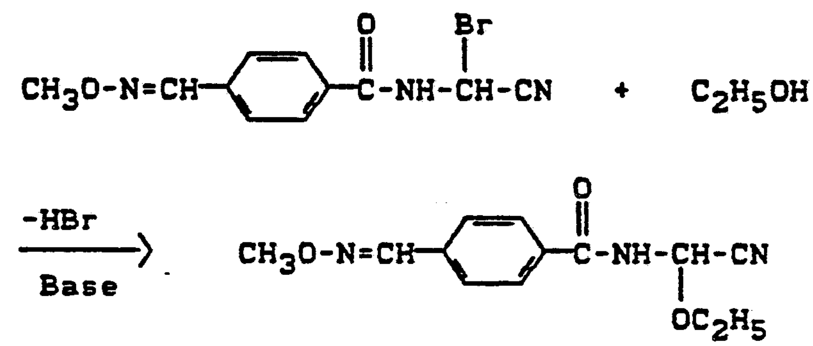


Verwendet man beispielsweise 2-(4-Methoximinomethylbenzamido)-2-methoxy-acetamid als Ausgangsverbindung und p-Toluolsulfonylchlorid als Dehydratisierungsmittel, so läßt sich der Reaktionsablauf des erfindungsgemäßen Verfahrens (b) durch das folgende Formelschema darstellen:

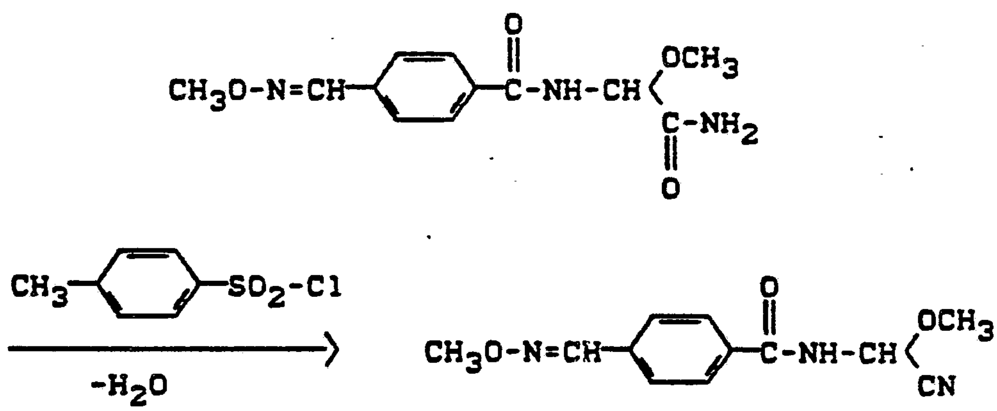

Verwendet man beispielsweise N-(Allyloxy-cyano-methyl)-4-methoximinomethyl-benzamid als Ausgangsverbindung und Schwefelwasserstoff als Reagenz, so läßt sich der Reaktionsablauf des erfindungsgemäßen Verfahrens (c) durch das folgende Formelschema darstellen:

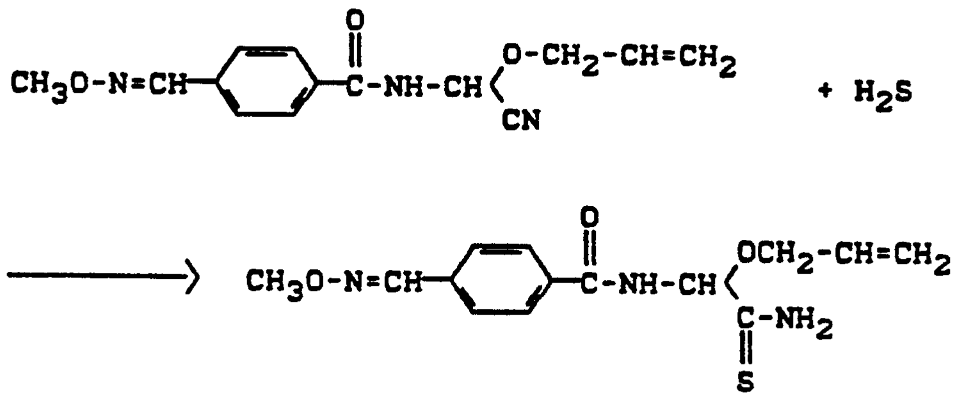

Verwendet man beispielsweise 4-Formyl-N-(cyano-2-furylmethyl)-benzamid und O-Methylhydroxylamin Hydrochlorid als Ausgangsstoffe, so läßt sich der Reaktionsablauf des erfindungsgemäßen Verfahrens (d) durch das folgende Formelschema darstellen:

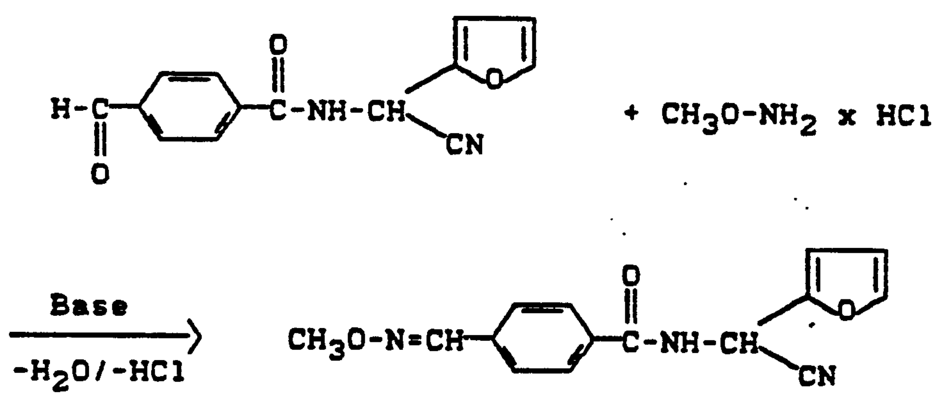

Verwendet man beispielsweise 4-Formylbenzoylchlorid und 2-Amino-2-furyl-acetonitril Hydrochlorid als Ausgangsstoffe, so läßt sich der Reaktionsablauf des erfindungsgemäßen Verfahrens (e) durch das folgende Formelschema darstellen:

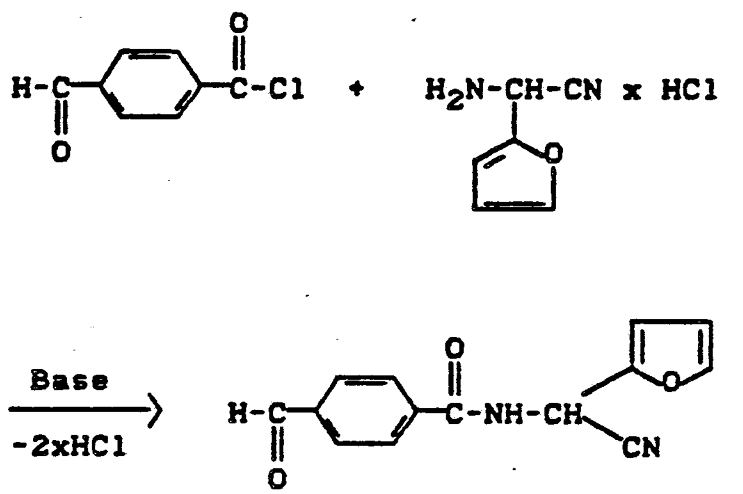


Die zur Durchführung des erfindungsgemäßen Verfahrens (a) als Ausgangsstoffe benötigten bromsubstituierten Benzamide sind durch die Formel (II) allgemein definiert. In dieser Formel (II) stehen X und n vorzugsweise für diejenigen Reste und Indices, die bereits im Zusammenhang mit der Beschreibung der erfindungsgemäßen Stoffe der Formel (I) für diesen Substituenten bzw. Index genannt wurden.

$R^{2-1}$ steht vorzugsweise für Cyano oder für einen Carbamoylrest.

Die bromsubstituierten Benzamide der Formel (II) sind noch nicht bekannt.

Man erhält sie, wenn man substituierte Benzoylchlorid-Verbindungen der Formel (V),

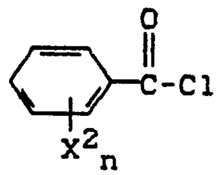

(V)

in welcher

$X^2$ für einen Alkanoylrest steht und

n die oben angegebene Bedeutung hat,

zunächst in einer 1. Stufe mit α-Aminoacetonitril Hydrochlorid der Formel (VII)

$$H_2N-CH_2-CN \quad x \quad HCl \qquad (VII)$$

gegebenenfalls in Gegenwart eines Verdünnungsmittels wie beispielsweise Dimethylformamid, und gegebenenfalls in Gegenwart eines Säurebindemittels, wie beispielsweise Triethylamin, bei Temperaturen zwischen -20° C und +50° C umsetzt und die so erhältlichen Benzoylaminoacetonitrile der Formel (VIIIa),

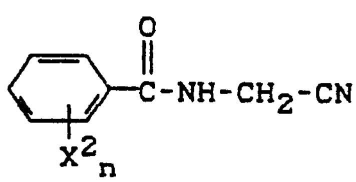

(VIIIa)

in welcher

$X^2$ und n die oben angegebene Bedeutung haben,

a) entweder direkt mit elementarem Brom, gegebenenfalls in Gegenwart eines Verdünnungsmittels, wie beispielsweise Essigsäureethylester, Tetrahydrofuran oder Essigsäure und gegebenenfalls in Gegenwart eines Katalysators, wie beispielsweise Bromwasserstoffsäure, bei Temperaturen zwischen -20° C und +50° C umsetzt oder

b) die Verbindungen der Formel (VIIIa) mit O-Alkylhydroxylaminen der Formel (IV),

$$R^3O\text{-}NH_2 \quad (IV)$$

in welcher

$R^3$ für Alkyl steht,

oder mit deren Säureadditionssalzen in Analogie zur Durchführung des erfindungsgemäßen Verfahrens (d) gegebenenfalls in Gegenwart eines Verdünnungsmittels, wie beispielsweise Dimethylformamid, und gegebenenfalls in Gegenwart eines Säurebindemittels, wie beispielsweise Triethylamin, bei Temperaturen zwischen -20° C und +50° C umsetzt und die so erhältlichen Benzoylaminoacetonitrile der Formel (VIIIb),

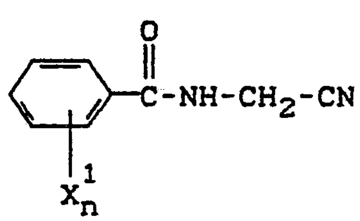

(VIIIb)

in welcher

$X^1$ für einen Alkoximinoalkylrest steht und

n die oben angegebene Bedeutung hat,

in einer weiteren Reaktionsstufe mit elementarem Brom, gegebenenfalls in Gegenwart eines Verdünnungsmittels wie beispielsweise Essigsäureethylester, Tetrahydrofuran oder Essigsäure und gegebenenfalls in Gegenwart eines Katalysators wie beispielsweise Bromwasserstoffsäure bei Temperaturen zwischen - 20° C und +50° C umsetzt.

Die substituierten Benzoylchlorid-Verbindungen der Formel (V) sind bekannt (vgl. z.B. Chem. Ber. 71, 335 - 341 [1938]; Bull. Soc. Chem. Fr. 1970, 4452 - 4459; DE-OS 29 13 770; EP 153 826) oder lassen sich in Analogie zu bekannten Verfahren auf einfache Art und Weise erhalten.

Das α-Aminoacetonitril Hydrochlorid der Formel (VII) und die Hydroxylamin-Derivate der Formel (IV) bzw. deren Säureadditionssalze sind allgemein bekannte Verbindungen der organischen Chemie.

Die zur Durchführung des erfindungsgemäßen Verfahrens (a) weiterhin als Ausgangsstoffe benötigten Alkohole oder Heterocyclen sind durch die Formel (III) allgemein definiert. In dieser Formel (III) steht $R^1$ vorzugsweise für diejenigen Reste, die bereits im Zusammenhang mit der Beschreibung der erfindungsgemäßen Stoffe der Formel (I) für diesen Substituenten genannt wurden.

Die Alkohole oder Heterocyclen der Formel (III) sind allgemein bekannte Verbindungen der organischen Chemie.

Die zur Durchführung des erfindungsgemäßen Verfahrens (b) als Ausgangsstoffe benötigten substituierten Benzamide sind durch die Formel (Ia1) allgemein definiert. In dieser Formel (Ia1) stehen $R^1$, X und n vorzugsweise für diejenigen Reste und Indices, die bereits im Zusammenhang mit der Beschreibung der erfindungsgemäßen Stoffe der Formel (I) für diese Substituenten und Indices genannt wurden.

Die substituierten Benzamide der Formel (Ia1) sind erfindungsgemäße Verbindungen und erhältlich mit Hilfe des erfindungsgemäßen Verfahrens (a).

Die zur Durchführung des erfindungsgemäßen Verfahrens (c) als Ausgangsstoffe benötigten substituierten Benzamide sind durch die Formel (Ib) allgemein definiert. In dieser Formel (Ib) stehen $R^1$, X und n vorzugsweise für diejenigen Reste und Indices, die bereits im Zusammenhang mit der Beschreibung der erfindungsgemäßen Stoffe der Formel (I) für diese Substituenten und Indices genannt wurden.

Die substituierten Benzamide der Formel (Ib) sind erfindungsgemäße Verbindungen und erhältlich mit Hilfe der erfindungsgemäßen Verfahren (a), (b), (d) oder (e).

Die zur Durchführung des erfindungsgemäßen Verfahrens (d) als Ausgangsstoffe benötigten substituierten Benzamide sind durch die Formel (If) allgemein definiert.

In dieser Formel (If) stehen $R^1$ und n vorzugsweise für diejenigen Reste und Indices, die bereits im Zusammenhang mit der Beschreibung der erfindungsgemäßen Stoffe der Formel (I) für diese Substituenten bzw. Indices genannt wurden.

$X^2$ steht vorzugsweise für geradkettiges oder verzweigtes Alkanoyl mit 1 bis 6 Kohlenstoffatomen, insbesondere für geradkettiges oder verzweigtes Alkanoyl mit 1 bis 4 Kohlenstoffatomen, besonders bevorzugt für Formyl oder Acetyl.

Die substituierten Benzamide der Formel (If) sind erfindungsgemäße Verbindungen und erhältlich mit Hilfe der erfindungsgemäßen Verfahren (a), (b) oder (e).

Die zur Durchführung des erfindungsgemäßen Verfahrens (d) weiterhin als Ausgangsstoffe benötigten Hydroxylamin-Derivate sind durch die Formel (IV) allgemein definiert. In dieser Formel (IV) steht $R^3$ vorzugsweise für geradkettiges oder verzweigtes Alkyl mit 1 bis 6, insbesondere 1 bis 4 Kohlenstoffatomen, besonders bevorzugt für Methyl.
Als Säureadditionssalze kommen insbesondere Hydrohalogenide wie z.B. Hydrochloride oder Hydrobromide infrage.

Die Hydroxylamin-Derivate der Formel (IV) und deren Säureadditionssalze sind allgemein bekannte Verbindungen der organischen Chemie.

Die zur Durchführung des erfindungsgemäßen Verfahrens (e) als Ausgangsstoffe benötigten substituierten

Benzoylchloride sind durch die Formel (V) allgemein definiert. In dieser Formel (V) stehen $X^2$ und n vorzugsweise für diejenigen Reste und Indices, die bereits im Zusammenhang mit der Beschreibung der erfindungsgemäßen Stoffe und Vorprodukte der Formel (If) für diese Substituenten bzw. Indices genannt wurden.

Die substituierten Benzoylchloride der Formel (V) sind bekannt (vgl. Literaturangaben auf S. 16).

Das zur Durchführung des erfindungsgemäßen Verfahrens (e) weiterhin als Ausgangsstoff benötigte Furanylaminoacetonitril ist durch die Formel (VI) definiert.

Das Furanylaminoacetonitril ist bekannt (vgl. BE 833 233 vom 09.03.1976 oder Can. PA.10 63 102 vom 25.09.1979).

Als Verdünnungsmittel zur Durchführung des erfindunsgemäßen Verfahrens (a) kommen inerte organische Lösungsmittel infrage.

Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide wie Dimethylformamid, Dimethylacetamid,

N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (a) wird vorzugsweise in Gegenwart eines geeigneten Säurebindemittels durchgeführt. Hierzu gehören vorzugsweise tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen -50° C und +100° C, vorzugsweise bei Temperaturen zwischen -30° C und +50° C.

Zur Durchführung des erfindungsgemäßen Verfahrens (a) setzt man pro Mol an bromsubstituiertem Benzamid der Formel (II) im allgemeinen 1.0 bis 3.0 Mol, insbesondere 1.0 bis 1.2 Mol an Alkohol oder Heterocyclus der Formel (III) und 1.0 bis 3.0 Mol, insbesondere 1.0 bis 2.0 Mol an Säurebindemittel ein.

In einer bevorzugten Durchführungsform stellt man die als Ausgangsstoffe verwendeten bromsubstituierten Benzamide der Formel (II) in einer vorgelagerten Reaktion direkt im Reaktionsgefäß her, indem man die entsprechenden Cyanmethylamide der Formel (VIII),

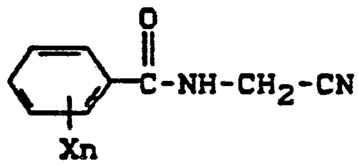

(VIII)

in welcher

X und n die oben angegebene Bedeutung haben,

mit einem geeigneten Bromierungsmittel, wie beispielsweise mit elementarem Brom, in einem geeigneten Verdünnungsmittel, wie beispielsweise Essigsäure oder Essigester, und gegebenenfalls Gegenwart eines geeigneten sauren Katalysators, wie beispielsweise Bromwasserstoffsäure, bei Temperaturen zwischen -20° C und + 20° C bromiert und direkt anschließend im 'Eintopfverfahren' gemäß dem erfindungsgemäßen Verfahren (a) weiter umsetzt.

Je nach Wahl des Verdünnungsmittels und in Abhängigkeit von der Säurekonzentration erhält man bei dieser vorgelagerten Bromierungsreaktion entweder eine gleichzeitige Hydratisierung der Nitrilgruppe zu den entsprechenden Amiden der Formel (IIa),

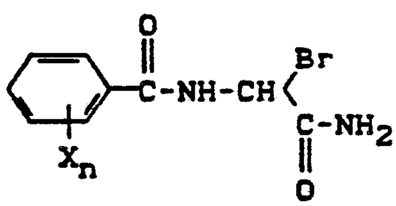

(IIa)

in welcher

X und n die oben angegebene Bedeutung haben,

oder Amide der Formel (IIb),

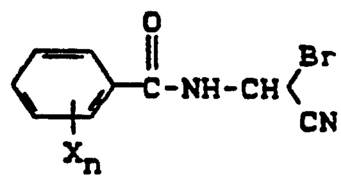

(IIb)

in welcher

X und n die oben angegebene Bedeutung haben,

bei denen die Nitrilgruppe als solche erhalten bleibt (vergl. EP 59 536, EP 135 304 sowie die Herstellungsbeispiele).

Die Durchführung des erfindungsgemäßen Verfahrens (a) sowie die Aufarbeitung und Isolierung der Reaktionsprodukte der Formel (Ia) erfolgt danach in Analogie zu bekannten Verfahren (vergl. EP 59 536).

Als Dehydratisierungsmittel zur Durchführung des erfindungsgemäßen Verfahrens (b) kommen alle üblichen wasserabspaltenden Mittel infrage. Mit besonderem Vorteil verwendet man Säurechloride oder Säureanhydride, wie beispielsweise Trifluoracetanhydrid, p-Toluolsulfonylchlorid, Trichloracetylchlorid, Methansulfonylchlorid, Titantetrachlorid oder Phosphoroxychlorid.

Als Verdünnungsmittel zur Durchführung des erfindungsgemäßen Verfahrens (b) kommen ebenfalls inerte organische Lösungsmittel infrage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Pyridin, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Nitrile, wie Acetonitril oder Propionitril oder Amide, wie Dimethylformamid.

Das erfindungsgemäße Verfahren (b) wird gegebenenfalls in Gegenwart eines geeigneten Säurebindemittels durchgeführt. Als solche eignen sich insbesondere tertiäre Amine, wie N-Methylmorpholin, Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (b) in einem größeren Bereich variiert werden. Im allgemeinen arbeitet man bei Temperaturen zwischen -20° C und +80° C, vorzugsweise bei Temperaturen zwischen 0° C und +40° C.

Zur Durchführung des erfindungsgemäßen Verfahrens (b) setzt man pro Mol substituiertem Benzamid der Formel (Ia1) im allgemeinen 1.0 bis 5.0 Mol, vorzugsweise 1.0 bis 3.0 Mol an Dehydratisierungsmittel ein. Die Reaktionsdurchführung, Aufarbeitung und Isolierung der Reaktionsprodukte der Formel (Ib) erfolgt in Analogie zu bekannten Verfahren (vergl. EP 59 536).

Als Verdünnungsmittel zur Durchführung des erfindungsgemä-

ßen Verfahrens (c) kommen ebenfalls inerte organische Lösungsmittel infrage. Hierzu gehören insbesondere die bei Verfahren (a) genannten organischen Lösungsmittel. Mit besonderem Vorteil verwendet man Toluol oder Pyridin als Verdünnungsmittel.

Das erfindungsgemäße Verfahren (c) wird vorzugsweise in Gegenwart eines basischen Katalysators durchgeführt. Als solche eignen sich insbesondere die bei Verfahren (a) aufgezählten tertiären Amine. Mit besonderem Vorzug verwendet man Triethylamin.

Die Reaktionstemperaturen können bei der Durchführung des erfindungsgemäßen Verfahrens (c) in einem größeren Bereich variiert werden. Im allgemeinen arbeitet man bei Temperaturen zwischen -20° C und +30° C, vorzugsweise bei Temperaturen zwischen 0° C und +20° C.

Zur Durchführung des erfindungsgemäßen Verfahrens (c) setzt man pro Mol substituiertem Benzamid der Formel (Ib) im allgemeinen 1.0 bis 5.0 Mol, insbesondere 1.0 bis 2.0 Mol an basischen Katalysator ein und leitet gasförmigen Schwefelwasserstoff durch die Reaktionslösung. Die Aufarbeitung und Isolierung der Reaktionsprodukte der Formel (Ic) erfolgt durch Abfiltrieren von ausgefallenem festem Produkt oder durch Entfernen der flüchtigen Bestandteile der Reaktionsmischung durch Eindampfen (vgl. auch EP 59 536).

Als Verdünnungsmittel zur Durchführung des erfindungsgemäßen Verfahrens (d) kommen vorzugsweise polare organische

Lösungsmittel oder deren Gemische mit Wasser infrage. Insbesondere verwendet man dipolare, aprotische Lösungsmittel wie beispielsweise Nitrile, wie Acetonitril oder Propionitril oder Amide, wie Dimethylformamid, Dimethylacetamid, N-Methylformanilid, N-Methylpyrrolidon oder Hexamethylphosphorsäuretriamid, gegebenenfalls auch in Mischung mit mit Wasser mischbaren Ethern, wie Dioxan oder Tetrahydrofuran, oder Alkohole, wie Methanol, Ethanol oder Propanol, sowie deren Gemische mit Wasser.

Das erfindungsgemäße Verfahren (d) wird gegebenenfalls in Gegenwart eines geeigneten Säurebindemittels durchgeführt.

Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate oder -acetate, wie Natriumcarbonat, Kaliumcarbonat, Natriumhydrogencarbonat oder Natriumacetat, sowie tertiäre Amine, wie Triethylamin, N,N-Dimethylanilin, Pyridin, N,N-Dimethylaminopyridin, Diazabicyclooctan (DABCO), Diazabicyclononen(DBN) oder Diazabicycloundecen(DBU).

Die Reaktionstemperaturen können bei der Durchführung des erfindungsgemäßen Verfahrens (d) in einem größeren Bereich variiert werden. Im allgemeinen arbeitet man bei Temperaturen zwischen -20° C und 100° C, vorzugsweise bei Temperaturen zwischen 0° C und 80° C.

Zur Durchführung des erfindungsgemäßen Verfahrens (d) setzt man pro Mol an substituiertem Benzamid der Formel (If) im allgemeinen 1,0 bis 1,5 Mol, vorzugsweise 1,0 bis 1,3 Mol an Hydroxylamin-Derivat der Formel (IV) und gegebenenfalls 1,0 bis 3,0 Mol, vorzugsweise 1,0 bis 2,0 Mol an Säurebindemittel ein. In einer bevorzugten Ausführungsform stellt man die als Ausgangsstoffe erforderlichen substituierten Benzamide der Formel (If) direkt im Reaktionsgefäß in einer vorgelagerten Reaktion gemäß dem erfindungsgemäßen Verfahren (e) her und setzt sie direkt aus der Reaktionsmischung heraus ohne Isolierung mit dem Hydroxylamin-Derivat der Formel (IV) weiter um. Die Reaktionsdurchführung, Aufarbeitung und Isolierung der Reaktionsprodukte der Formel (Id) erfolgt in Analogie zu allgemein üblichen Verfahren (vgl. auch die Herstellungsbeispiele).

Als Verdünnungsmittel zur Durchführung des erfindungsgemäßen Verfahrens (e) kommen inerte organische Lösungsmittel infrage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl oder -diethylether, Ketone wie Aceton oder Butanon, Nitrile, wie Acetonitril oder Propionitril, Amide wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (e) wird gegebenenfalls in Gegenwart eines geeigneten Säurebindemittels durchgeführt.

Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen(DBN) oder Diazabicycloundecen (DBU).

Die Reaktionstemperaturen können bei der Durchführung des erfindungsgemäßen Verfahrens (e) in einem größeren Bereich variiert werden. Im allgemeinen arbeitet man bei Temperaturen zwischen -20°C und +100°C, vorzugsweise bei Temperaturen zwischen 0°C und 50°C.

Zur Durchführung des erfindungsgemäßen Verfahrens (e) setzt man pro Mol an substituiertem Benzoylchlorid der Formel (V) im allgemeinen 1,0 bis 1,5 Mol vorzugsweise äquimolare Mengen an Furanylaminoacetonitril und gegebenenfalls 1,0 bis 2,0 Mol an Säurebindemittel ein. Die

Reaktionsdurchführung, Aufarbeitung und Isolierung der Reaktionsprodukte der Formel (Ie) erfolgt in Analogie zu allgemein bekannten Verfahren.

Die erfindungsgemäßen Wirkstoffe weisen eine Wirkung gegen Schädlinge auf und können zur Bekämpfung von unerwünschten Schadorganismen praktisch eingesetzt werden. Die Wirkstoffe sind für den Gebrauch, z.B. als Pflanzenschutzmittel z.B. als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;

Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Pflanzenkrankheiten im Gemüseanbau, wie beispielsweise gegen den Erreger der Tomatenbraunfäule (Phytophthora infestans) einsetzen. Besonders hervorzuheben ist, daß die erfindungsgemäßen Wirkstoffe nicht nur protektiv wirksam sind, sondern darüberhinaus auch kurative Eigenschaften besitzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Al-

kylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Ver-

streuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Herstellungsbeispiele

Beispiel 1:

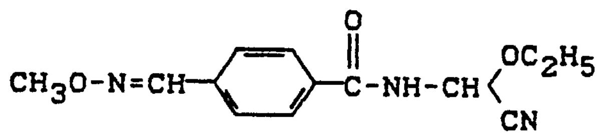

(Verfahren a)

Zu 10 g (0,046 Mol) 2-(4-Methoximinomethylbenzoyl)-amino-acetonitril in 140 ml Essigsäureethylester und 140 ml Tetrahydrofuran gibt man 7,35 g (0,046 Mol) Brom sowie einige Tropfen einer 33-prozentigen Bromwasserstofflösung in Eisessig und wärmt vorsichtig an. Bei ca. 30°C setzt die Bromierungsreaktion ein. Nach beendeter Reaktion kühlt man auf -40°C ab und gibt eine Mischung aus 9 g (0,195 Mol) Ethanol, 9,3 g (0,092 Mol) Triethylamin und 9 g Essigsäureethylester zu, erwärmt auf 0°C, filtriert ausgefallenes Triethylaminhydrobromid ab und engt im Vakuum ein. Das Produkt kristallisiert aus Cyclohexan.

Man erhält 9,1 g (76 % der Theorie) an 2-Ethoxy-2-[(4-methoximinomethylbenzoyl)-amino-acetonitril vom Schmelzpunkt 143°C - 145°C.

Beispiel 2:

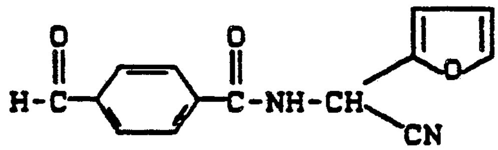

(Verfahren e)

Zu 4,75 g (0,03 Mol) 2-Amino-2-furyl-acetonitril Hydrochlorid (vgl. BE 833 233) in 40 ml Dimethylformamid gibt man unter Eiskühlung zunächst 8,4 ml (0,06 Mol) Triethylamin und danach tropfenweise eine Lösung von 5,05 g (0,03 Mol) 4-Formylbenzoylchlorid in 15 ml Tetrahydrofuran. Nach beendeter Zugabe rührt man 16 Stunden bei Raumtemperatur, gießt dann die Reaktionsmischung in Eiswasser und extrahiert mit Essigsäureethylester. Die vereinigten organischen Phasen werden mit gesättigter wässriger Kochsalzlösung gewaschen, über Natriumsulfat getrocknet und im Vakuum eingeengt. Das zurückbleibende Öl wird chromatographisch gereinigt (Kieselgel/ Dichlormethan).
Man erhält 3,4 g (45 % der Theorie) an 2-[(4-Formylbenzoyl)-amino]-2-furylacetonitril als Öl.

$^1$H-NMR ($CDCl_3$/Tetramethylsilan): $\delta$ = 6,35 (d) ppm.

Beispiel 3:

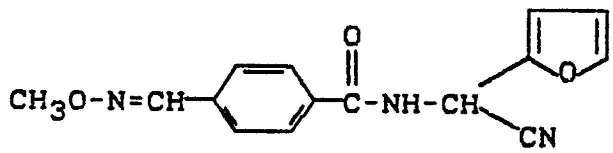

(Verfahren d/Verfahren e "Eintopfreaktion")

Zu 4,7 g (0,0297 Mol) 2-Amino-2-furyl-acetonitril Hydrochlorid in 50 ml Dimethylformamid gibt man unter Eiskühlung zunächst 6,0 g (0,0593 Mol) Triethylamin und anschließend tropfenweise eine Lösung von 5,0 g (0,0297 Mol) 4-Formylbenzoylchlorid in 15 ml Tetrahydrofuran. Nach beendeter Zugabe rührt man 15 Minuten bei Raumtemperatur und gibt dann nacheinander 2,5 g (0,0299 Mol) O-Methylhydroxylamin und 3,0 g (0,0297 Mol) Triethylamin zu und rührt weitere 16 Stunden bei Raumtemperatur. Zur Aufarbeitung gießt man die Reaktionsmischung in Wasser und extrahiert mehrfach mit Essigsäureethylester. Die vereinigten organischen Phasen werden mit Wasser gewaschen, über Natriumsulfat getrocknet und im Vakuum vom Lösungsmittel befreit. Der Rückstand wird aus Toluol umkristallisiert.

Man erhält 4,19 g (49 % der Theorie) an 2-Furyl-2-[(4-methoximinomethylbenzoyl)-amino]-acetonitril vom Schmelzpunkt 143$^{\circ}$ C.

In entsprechender Weise und gemäß den allgemeinen Angaben zur Herstellung erhält man die folgenden substituierten Benzamide der allgemeinen Formel (I):

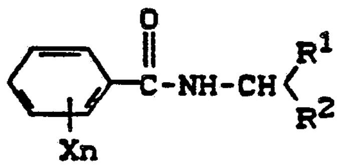
(I)

| Bsp. Nr. | $R^1$ | $R^2$ | 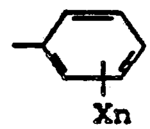 | Schmelzpunkt/[°C] |
|---|---|---|---|---|
| 4 | $CH_3O$ | CN | 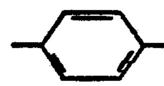 $CH=N-OCH_3$ | 117-120 |
| 5 | $CH_3-(CH_2)_2-O-$ | CN | 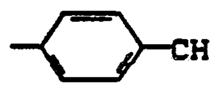 $CH=N-OCH_3$ | 115-117 |
| 6 | $CH_3-(CH_2)_3-O-$ | CN | 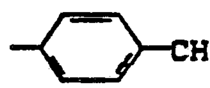 $CH=N-OCH_3$ | 104-105 |
| 7 | $CH_2=CH-CH_2-O-$ | CN | 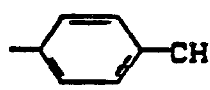 $CH=N-OCH_3$ | 111-114 |
| 8 | $HC\equiv C-CH_2-O-$ | CN | 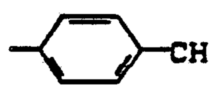 $CH=N-OCH_3$ | 101-104 |
| 9 | 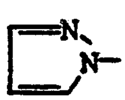 | CN | 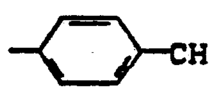 $CH=N-OCH_3$ | 165-167 |
| 10 | $HC\equiv C-(CH_2)_2-O-$ | CN | 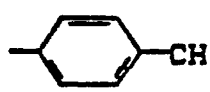 $CH=N-OCH_3$ | 102-105 |

| Bsp. Nr. | $R^1$ | $R^2$ | 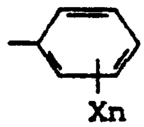 | Schmelz-punkt/[°C] |
| --- | --- | --- | --- | --- |
| 11 | $CH_3-C≡C-CH_2-O-$ | CN | 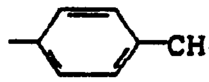 $-CH=N-OCH_3$ | 76-80 |
| 12 | $CH_2=CH-CH-O-$<br>\|<br>$CH_3$ | CN | 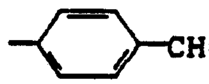 $-CH=N-OCH_3$ | 83-86 |
| 13 | $HC≡C-CH-O-$<br>\|<br>$CH_3$ | CN | 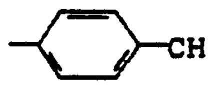 $-CH=N-OCH_3$ | |
| 14 | $CH_3-CH_2-CH_2-O-$ | CN | 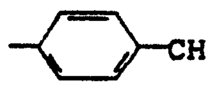 $-CH=N-O(CH_2)_5CH_3$ | 96-97 |
| 15 | $HC≡C-CH_2-O-$ | CN | 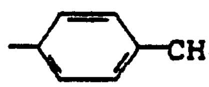 $-CH=N-O(CH_2)_5CH_3$ | 54-56 |
| 16 | $H_2C=CH-CH_2-O-$ | CN | 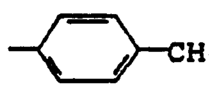 $-CH=N-O(CH_2)_5CH_3$ | 78-79 |
| 17 | 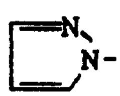 | CN | 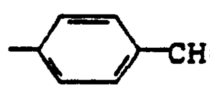 $-CH=N-O(CH_2)_5CH_3$ | 121-123 |

Herstellung der Ausgangsverbindungen

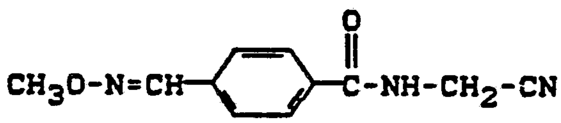

(VIII-1)

Zu 5,6 g (0,06 Mol) 2-Aminoacetonitril Hydrochlorid in 50 ml Dimethylformamid gibt man bei 0° C 12,2 g (0,12 Mol) Triethylamin und danach bei 25° C tropfenweise unter Rühren 10,1 g (0,06 Mol) 4-Formylbenzoylchlorid in 10 ml Tetrahydrofuran. Nach beendeter Zugabe rührt man 15 Minuten bei Raumtemperatur und setzt dann zunächst eine Lösung von 5 g (0,06 Mol) O-Methylhydroxylamin Hydrochlorid in 30 ml Dimethylformamid und danach 6,1 g (0,06 Mol) Triethylamin zu. Darauf rührt man weitere 12 Stunden bei Raumtemperatur, gießt dann das Reaktionsgemisch in 800 ml Eiswaser, filtriert das Produkt ab, wäscht mit Wasser und trocknet.

Man erhält 11,5 g (88,2 % der Theorie) an 2-(4-Methoximinobenzoyl)-amino-acetonitril vom Schmelzpunkt 191° - 193° C.

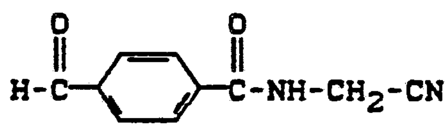

(VIII-2)

Zu 5,6 g (0,06 Mol) 2-Aminoacetonitril Hydrochlorid in 50 ml Dimethylformamid gibt man bei 0° C zunächst 12,2 g (0,12 Mol) Triethylamin und danach tropfenweise unter

Rühren eine Lösung von 10,1 g (0,06 Mol) 4-Formylbenzoylchlorid in 10 ml Tetrahydrofuran, so daß die Innentemperatur 10° C nicht übersteigt. Nach beendeter Zugabe rührt man weitere 15 Minuten bei Raumtemperatur, gießt den Ansatz in 400 ml Eiswasser filtriert und trocknet das so erhältliche Produkt. Extraktion des Filtrates mit 5 mal 100 ml Essigsäureethylester, Waschen der vereinigten organischen Phasen, Trocknen über Natriumsulfat und Einengen im Vakuum liefert eine weitere Fraktion. Insgesamt erhält man 8,9 g (79 % der Theorie) an 2-(4-Formylbenzoyl)-amino-acetonitril vom Schmelzpunkt 122° -125° C.

Anwendungsbeispiele

In dem folgenden Anwendungsbeispiel wurde die nachstehend aufgeführte Verbindung als Vergleichssubstanz eingesetzt:

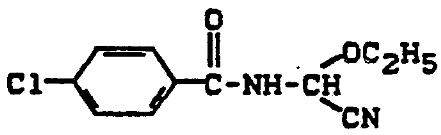

(A)

2-(4-Chlorbenzoylamino)-2-ethoxy-acetonitril (bekannt aus EP 59 536).

Beispiel A

Phytophthora-Test (Tomate)/kurativ

Lösungsmittel: 4,7 Gewichtsteile Aceton
Emulgator: 0,3 Gewichtsteile Alkylaryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer wäßrigen Sporensuspension von Phytophthora infestans inokuliert. Die Pflanzen verbleiben 7 Stunden bei 20°C und 100 % relativer Luftfeuchtigkeit in einer Inkubationskabine. Nach einer kurzen Abtrocknungszeit werden die Pflanzen mit der Wirkstoffzubereitung tropfnaß gespritzt.

Die Pflanzen werden in einer Inkubationskabine mit 100% relativer Luftfeuchtigkeit und ca. 20°C aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindungen gemäß den Herstellungsbeispielen: 1, 4, 5, 7.

Beispiel B

Pflanzenverträglichkeitstest

Testpflanze: Tomate
Versuchsdauer: 6 Tage
Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Mit dieser Wirkstoffzubereitung bespritzt man junge Pflanzen bis zur Tropfnässe und stellt sie in einem Gewächshaus bei ca. 20° C auf.

Die Pflanzen werden auf Schäden wie Wuchsbeeinträchtigungen, Verfärbungen und Nekrosen ausgewertet. Angegeben wird der Schädigungsgrad der Pflanzen in %. Dabei bedeutet:

0 %: keine Schäden
100 %: Pflanze vollkommen geschädigt.

Eine deutliche Überlegenheit in der Pflanzenverträglichkeit gegenüber dem Stand der Technik zeigt bei diesem Test z.B. die Verbindung gemäß dem Herstellungsbeispiel: 7

Patentansprüche

1. Substituierte Benzamide der Formel (I),

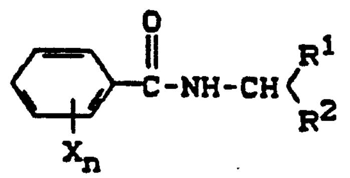

(I)

in welcher

$R^1$ für Alkoxy, Alkenyloxy, Alkinyloxy oder für einen Heterocyclylrest steht,

$R^2$ für Cyano, für einen Carbamoyl- oder für einen Thiocarbamoylrest steht,

X für einen Alkanoyl- oder für einen Alkoximinoalkylrest steht und

n für eine Zahl 1, 2 oder 3 steht.

2. Substituierte Benzamide gemäß Anspruch 1, wobei in der Formel (I)

$R^1$ für geradkettiges oder verzweigtes Alkoxy mit 1 bis 8 Kohlenstoffatomen, für jeweils geradkettiges oder verzweigtes Alkenyloxy oder Alkinyloxy mit jeweils 3 bis 8 Kohlenstoffatomen oder für einen 5- oder 6-gliedrigen Heterocyclylrest mit 1 bis 4 Heteroatomen steht,

$R^2$ für Cyano, für einen Carbamoyl- oder für einen Thiocarbamoylrest steht,

X für einen geradkettigen oder verzweigten Alkanoylrest mit 1 bis 6 Kohlenstoffatomen oder für einen geradkettigen oder verzweigten Alkoximinoalkylrest mit jeweils 1 bis 6 Kohlenstoffatomen in den einzelnen Alkylteilen steht und

n für eine Zahl 1, 2 oder 3 steht.

3. Substituierte Benzamide gemäß Anspruch 1, wobei in der Formel I

$R^1$ für geradkettiges oder verzweigtes Alkoxy mit 1 bis 6 Kohlenstoffatomen, für jeweils geradkettiges oder verzweigtes Alkenyloxy oder Alkinyloxy mit jeweils 3 bis 6 Kohlenstoffatomen oder für einen 1-Pyrazolyl-, 1-Imidazolyl-, 1-(1,2,4-Triazolyl)-, 1-Tetrazolyl- oder 2-Furylrest steht,

$R^2$ für Cyano, für einen Carbamoyl- oder für einen Thiocarbamoylrest steht,

X für einen geradkettigen oder verzweigten Alkanoylrest mit 1 bis 4 Kohlenstoffatomen oder für einen geradkettigen oder verzweigten Alkoximinoalkylrest mit jeweils 1 bis 4 Kohlenstoffatomen in den einzelnen Alkylteilen steht und

n für eine Zahl 1 oder 2 steht.

4. Substituierte Benzamide gemäß Anspruch 1, wobei in der Formel (I)

$R^1$ für Methoxy, Ethoxy, n- oder i-Propoxy, n-, i-, s-oder t-Butoxy, für Allyloxy, n- oder i-Butenyloxy, Propargyloxy, n- oder i-Butinyloxy, für einen 2-Furylrest oder für einen 1-Pyrazolylrest steht,

$R^2$ für Cyano steht,

X für Formyl, Acetyl oder Methoximinomethyl steht und

n für 1 steht.

5. Verfahren zur Herstellung von substituierten Benzamiden der allgemeinen Formel (I)

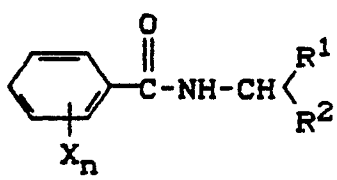
(I)

in welcher

$R^1$ für Alkoxy, Alkenyloxy, Alkinyloxy oder für einen Heterocyclylrest steht,

$R^2$ für Cyano, für einen Carbamoyl- oder für einen Thiocarbamoylrest steht,

X für einen Alkanoyl- oder für einen Alkoximinoalkylrest steht und

n für eine Zahl 1, 2 oder 3 steht,

dadurch gekennzeichnet, daß man

(a) substituierte Benzamide der Formel (Ia),

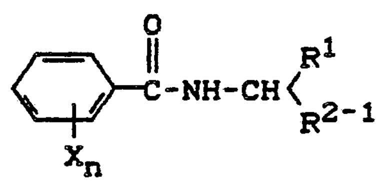

(Ia)

in welcher

$R^1$, X und n die oben angegebene Bedeutung haben und

$R^{2-1}$ für Cyano oder für einen Carbamoylrest steht,

erhält wenn man bromsubstituierte Benzamide der Formel (II),

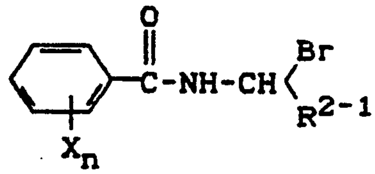

(II)

in welcher

$R^{2-1}$, X und n die oben angegebene Bedeutung haben,

mit Alkoholen oder Heterocyclen der Formel (III),

$R^1$-H (III)

in welcher

$R^1$ die oben angegebene Bedeutung hat,

gegebenenfalls in Gegenwart eines Verdünnungsmittels und gegebenenfalls in Gegenwart eines Säurebindemittels umsetzt;

(b) man erhält substituierte Benzamide der Formel (Ib),

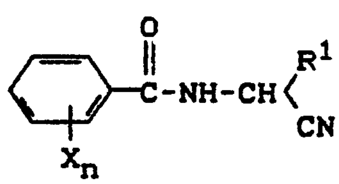

(Ib)

in welcher

$R^1$, X und n die oben angegebene Bedeutung haben,

erhält, wenn man die nach Verfahren (a) erhältlichen substituierten Benzamide der Formel (Ia1),

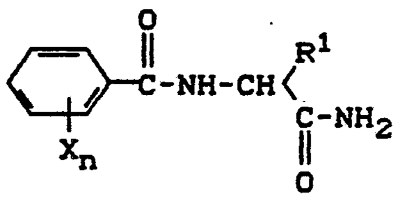

(Ia1)

in welcher

$R^1$, X und n die oben angegebene Bedeutung haben,

gegebenenfalls in Gegenwart eines Verdünnungsmittels und gegebenenfalls in Gegenwart eines Säurebindemittels mit einem wasserabspaltenden Reagenz dehydratisiert oder

(c) substituierte Benzamide der Formel (Ic),

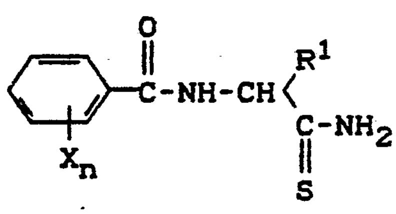

(Ic)

in welcher

$R^1$, X und n die oben angegebene Bedeutung haben,

erhält, wenn man die nach Verfahren (a), (b), (d) oder (e) erhältlichen substituierten Benzamide der Formel (Ib),

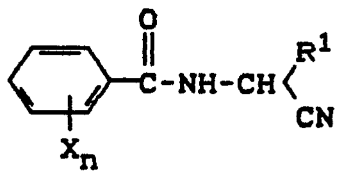

(Ib)

in welcher

$R^1$, X und n die oben angegebene Bedeutung haben,

mit Schwefelwasserstoff, gegebenenfalls in Gegenwart eines Verdünnungsmittels und gegebenenfalls in Gegenwart eines basischen Katalysators umsetzt oder

(d) substituierte Benzamide der Formel (Id),

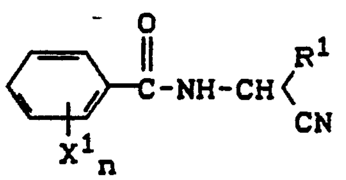

(Id)

in welcher

$R^1$ und n die oben angegebene Bedeutung haben und

$X^1$ für einen Alkoximinoalkylrest steht,

erhält, wenn man die nach Verfahren (a), (b) oder (e) erhältlichen substituierten Benzamide der Formel (If),

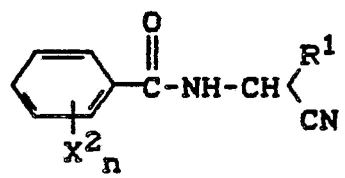

(If)

in welcher

$R^1$ und n die oben angegebene Bedeutung haben und

$X^2$ für einen Alkanoylrest steht,

mit Hydroxylamin-Derivaten der Formel (IV),

$R^3O-NH_2$ (IV)

in welcher

$R^3$ für Alkyl steht,

oder mit deren Säureadditionssalzen,

gegebenenfalls in Gegenwart eines Verdünnungsmittels und gegebenenfalls in Gegenwart eines Säurebindemittels umsetzt oder

(e) substituierte Benzamide der Formel (Ie),

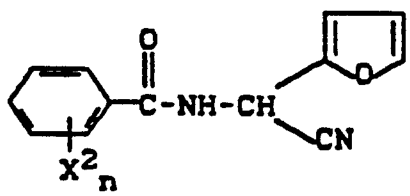

(Ie)

in welcher

$X^2$ und n die oben angegebene Bedeutung haben,

erhält, wenn man substituierte Benzoylchloride der Formel (V),

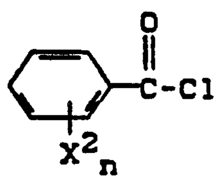

(V)

in welcher

$X^2$ und n die oben angegebene Bedeutung haben,

mit Furanylaminoacetonitril der Formel (VI)

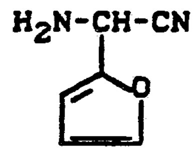

(VI)

oder dessen Säureadditionssalzen gegebenenfalls in Gegenwart eines Verdünnungsmittels und gegebenenfalls in Gegenwart eines Säurebindemittels umsetzt.

6. Schädlingsbekämpfungsmittel, gekennzeichnet durch einen Gehalt an mindestens einer Verbindung der allgemeinen Formel (I) gemäß den Ansprüchen 1 und 5.

7. Verfahren zur Bekämpfung von Schädlingen, dadurch gekennzeichnet, daß man mindestens eine Verbindung der Formel (I) gemäß den Ansprüchen 1 und 5 auf Schädlinge und/oder ihren Lebensraum einwirken läßt.

8. Verwendung von Verbindungen der allgemeinen Formel (I) gemäß den Ansprüchen 1 und 5 zur Bekämpfung von Schädlingen.

9. Verfahren zur Herstellung von Schädlingsbekämpfungmitteln, dadurch gekennzeichnet, daß man Verbindungen der allgemeinen Formel (I) gemäß den Ansprüchen 1 und 5 mit Streckmitteln und/oder oberflächenaktiven Mitteln vermischt.

10. Bromsubstituierte Benzamide der allgemeinen Formel (II),

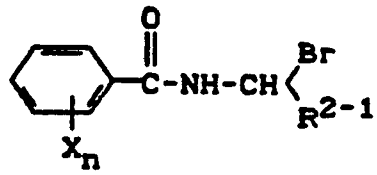

(II)

in welcher

$R^{2-1}$ für Cyano oder für einen Carbamoylrest steht,

X für einen Alkanoyl- oder für einen Alkoximinoalkylrest steht und

n für eine Zahl 1, 2 oder 3 steht.